# EUROPÄISCHE PATENTSCHRIFT

(11) **EP 1 370 175 B1**
(45) Veröffentlichungstag und Bekanntmachung des Hinweises auf die Patenterteilung: **04.10.2006**
(21) Anmeldenummer: 02726153.6
(22) Anmeldetag: 09.03.2002
(51) Int. Cl.: A61B 1/00, A61B 1/12, G02B 23/24, G02B 23/16

(54) **OPTISCHES INSTRUMENT, INSBESONDERE ENDOSKOPISCHES INSTRUMENT**
OPTICAL INSTRUMENT, ESPECIALLY ENDOSCOPIC INSTRUMENT
INSTRUMENT OPTIQUE, EN PARTICULIER INSTRUMENT ENDOSCOPIQUE

(30) Priorität: 20.03.2001 DE 10113365
(43) Veröffentlichungstag der Anmeldung: 17.12.2003
(73) Patentinhaber: Karl Storz GmbH & Co., 78532 Tuttlingen (DE)
(72) Erfinder: HUBER, Matthias, 78575 Emmingen (DE); KEHR, Ulrich, 70771 Leinfelden - Echterdingen (DE)
(74) Vertreter: Hofmeister, Frank
(86) Internationale Anmeldenummer: PCT/EP2002/002601
(87) Internationale Veröffentlichungsnummer: WO 2002/074159

(56) Entgegenhaltungen:
- US-A- 6 077 220

## Beschreibung

Die Erfindung betrifft ein Optisches Instrument, insbesondere endoskopisches Instrument, mit einem Gehäuse, in dem mindestens ein optisches System sowie eine hygroskopische Substanz angeordnet sind, wobei am Gehäuse eine Okularmuschel lösbar festgelegt ist.

Optische Instrumente und insbesondere in der Medizintechnik eingesetzte optische Instrumente, wie beispielsweise endoskopische Instrumente, sind prinzipiell fluiddichte Systeme. Durch eine Reihe von Gründen besteht aber die Möglichkeit, daß Feuchtigkeit in das Gehäuse eindringen kann, was zu einer die Sicht verschlechternden Trübung der optischen Systeme führen kann. Probleme können beispielsweise bereits bei der Fertigung der Instrumente in normaler Atmosphäre entstehen, wenn sich die Restfeuchte der Atmosphärenluft im Gehäuseinneren niederschlägt. Weiterhin kann Feuchtigkeit durch geringfügige Undichtigkeiten an Verbindungsstellen eindringen, an denen das Instrument zu Wartungs-, Reparatur- oder Montagezwecken auseinandernehmbar ist. Eine weitere starke Belastung der optischen Instrumente stellt das Reinigen mittels Autoklavieren dar, bei dem das Instrument unter wechselndem Druck Heißdampf bei etwa 140° C ausgesetzt wird. Diese Temperaturbelastung kann zu feinen Rissen führen, durch die wiederum Feuchtigkeit in das Gehäuse eindringen kann.

Zur Vermeidung solcher Beschlagprobleme durch sich auf dem optischen System niederschlagende Feuchtigkeit ist es bei optischen Instrumenten bekannt, im Gehäuse eine hygroskopische Substanz anzuordnen, die die im Gehäuseinnenraum anfallende Feuchtigkeit bindet, bevor diese sich auf dem mindestens einen optischen System niederschlägt.

Aus der US 6 077 220 A ist es beispielsweise bekannt, die hygroskopische Substanz in Kugel- oder Stabform lose im Gehäuse anzuordnen. Dies hat jedoch den Nachteil, daß beim Bewegen des Instruments Geräusche auftreten und zudem durch die Bewegung Abrieb der hygroskopischen Substanz erzeugt wird, der sich als Staub auf die optischen Systeme legen kann.

Aus der DE 37 08 124 C2 ist ein Endoskop bekannt, bei dem die hygroskopische Substanz als flexibles Streifenmaterial ausgebildet ist, die in einem speziell hierfür vorgesehenen Teil der Endoskopoptik zwischen dem Linsenträger und der Gehäuseaußenwand angeordnet ist. Diese bekannte Ausbildung hat den Nachteil, daß ein Austausch des hygroskopischen Streifenmaterials nur bei einer vollständigen Zerlegung des Endoskops möglich ist, was in der Regel nur im Herstellerwerk vorgenommen wird. Das Auswechseln der hygroskopischen Substanz ist bei diesem Instrument somit mit einem hohen Zeit- und Kostenaufwand verbunden.

Die DE 199 13 761 A1 offenbart eine Trocknungsvorrichtung, bei der ein Trocknungsmittel (hygroskopische Substanz) in ein formbares flächenförmiges Matrixmaterial eingebettet ist.

In der DE 195 07 205 A1 wird vorgeschlagen, die hygroskopische Substanz unter einem abnehmbaren Wandstück des Gehäuses anzuordnen, das mit einer Kupplung gasdicht mit der übrigen Gehäusewand verbunden ist. Diese Ausführungsform ermöglicht zwar ein schnelles und einfaches Auswechseln der hygroskopischen Substanz, jedoch unter Inkaufnahme des Nachteils, daß das Endoskop eine weitere Öffnung aufweist, deren Fluiddichtigkeit gewährleistet werden muß.

Davon ausgehend liegt der Erfindung die **Aufgabe** zugrunde, ein optisches Instrument der eingangs genannten Art so auszugestalten, daß die hygroskopische Substanz einfach und sicher in das Gehäuse integrierbar ist.

Die erfindungsgemäße **Lösung** dieser Aufgabenstellung ist dadurch gekennzeichnet, daß die hygroskopische Substanz in ein formbares Matrixmaterial eingebettet ist, und daß das mit der hygroskopischen Substanz versetzte Matrixmaterial auswechselbar in die Okularmuschel einsetzbar ist.

Durch die erfindungsgemäße Einbettung der hygroskopischen Substanz in ein frei formbares Matrixmaterial, das auswechselbar in die Okularmuschel einsetzbar ist, ist es erstmalig möglich, die hygroskopische Substanz sicher, orts- und abriebfest so anzuordnen, daß die hygroskopische Substanz einfach und schnell auswechselbar in dem Gehäuse angeordnet ist. Da die Substanz Bestandteil des durch das Matrixmaterial geformten Bauteils ist, entfällt eine separate Montage mit zusätzlichen Halteelementen, wie dies aus dem Stand der Technik bekannt ist.

Um einerseits die Feuchtigkeitsaufnahme durch die eingebettete hygroskopische Substanz zu verstärken und andererseits die freie Formbarkeit zu beliebigen Geometrien zu gewährleisten, wird vorgeschlagen, daß das Matrixmaterial im ausgehärteten Zustand elastisch und feuchtigkeitsdurchlässig ist. Als erfindungsgemäße Matrixmaterialien, die diese Eigenschaften aufweisen und die darüber hinaus auch durch Spritzgießen herstellbar sind, können beispielsweise Elastomere auf Silikon- und/oder Polyurethanbasis verwendet werden.

Durch Spritzgießen oder andere Formverfahren ist es so beispielsweise möglich, das mit der hygroskopischen Substanz versetzte Matrixmaterial als in die Okularmuschel des optischen Instruments einsetzbare zylindrische Hülse oder als in das Gehäuse des optischen Instruments einsetzbaren O-Ring auszubilden.

Gemäß einer vorteilhaften Ausgestaltungsform der Erfindung wird weiterhin vorgeschlagen, daß die Feuchtigkeitsbeladung der hygroskopischen Substanz optisch, beispielsweise durch eine Farbänderung der Substanz, anzeigbar ist. Die Verwendung einer hygroskopischen Substanz mit einer solchen Indikatoreigenschaft ist besonders vorteilhaft, da der Benutzer des Instruments, beispielsweise durch ein Kontrollfenster den Zustand der hygroskopischen Substanz kontrollieren kann, um diese bei Bedarf mitsamt dem formgebenden Matrixmaterial auszuwechseln.

Als Materialien für die hygroskopische Substanz können beispielsweise Silika-Gel oder poröse Keramiken, sogenannte Molekularsiebe, verwendet werden.

Schließlich wird mit der Erfindung vorgeschlagen, daß die hygroskopische Substanz aus einer Mischung verschiedener hygroskopischer Substanzen besteht. Auf diese Weise lassen sich bei Bedarf die unterschiedlichen Eigenschaften der Substanzen kombinieren, um eine bestmögliche hygroskopische Substanz für den bestimmten Einsatzzweck zu erhalten.

Weitere Merkmale und Vorteile der Erfindung ergeben sich anhand der nachfolgenden Beschreibung der zugehörigen Zeichnung, in der zwei Ausführungsbeispiele eines erfindungsgemäßen optischen Instruments nur beispielhaft schematisch dargestellt sind. In der Zeichnung zeigt:
- Fig. 1: einen Längsschnitt durch eine erste Ausführungsform eines erfindungsgemäßen optischen Instruments und
- Fig. 2: einen Längsschnitt durch eine zweite Ausführungsform eines erfindungsgemäßen optischen Instruments.

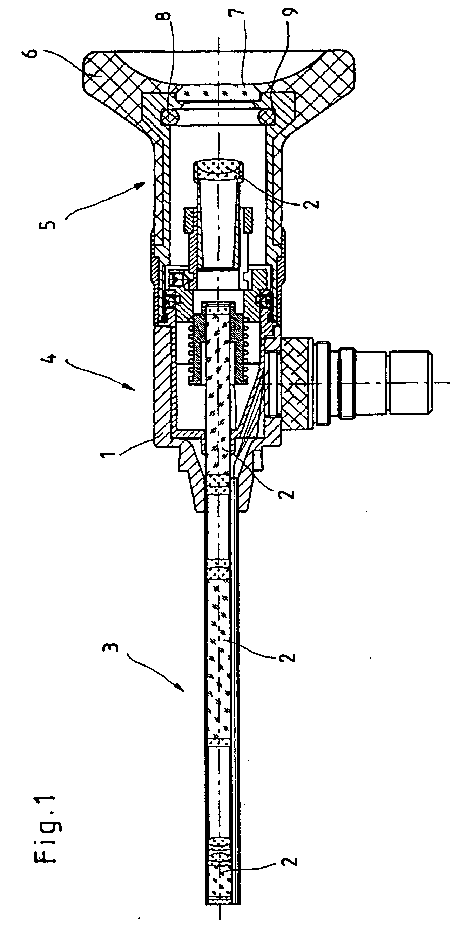

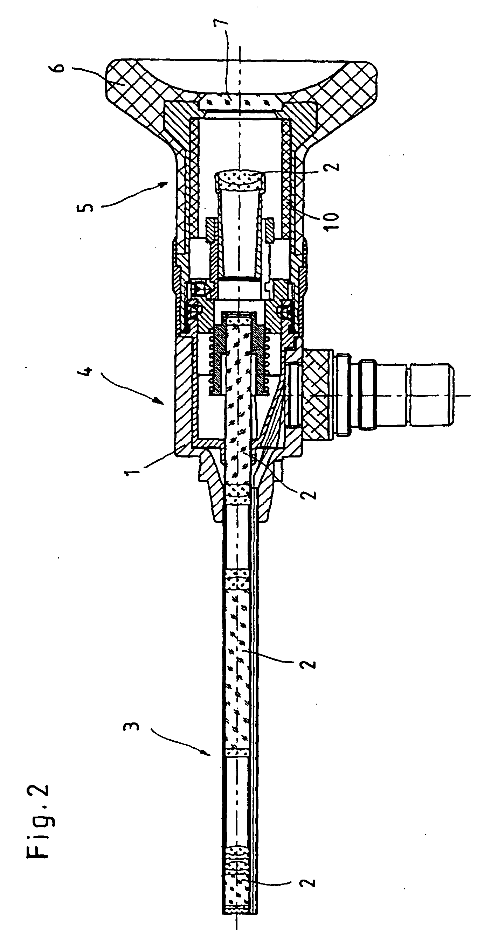

Bei dem in den Abbildungen Fig. 1 und 2 dargestellten optischen Instrument handelt es sich um ein starres Endoskop mit einem Gehäuse 1 zur Aufnahme optischer Systeme 2, in diesem Fall eines Linsensystems.

Das Gehäuse 1 besteht aus einem dünnen distalseitigen Schaft 3 und einem proximalseitigen Handhabungsteil 4 größeren Durchmessers. Im Inneren des fluiddicht mit dem Schaft 3 verbundenen Handhabungsteits 4 ist eine Okulareinheit 5 angeordnet. Zum Betrachten des über das optische System 2 zur Okulareinheit 5 weitergeleiteten Bildes, ist am proximalen Ende des Handhabungsteils 4 eine Okularmuschel 6 angeordnet, durch die der Benutzer des Endoskops blend- und störlichtfrei die Abbildung begutachten kann. Die Okularmuschel 6 ist einerseits mit dem Gehäuse 1 verschraubt und andererseits über ein beispielsweise eingekittetes Okulardeckglas 7 fluiddicht verschlossen.

Zum Entfernen der eventuell im Gehäuse 1 vorhandenen Restfeuchte sowie zur Aufnahme von über Undichtigkeiten in das Gehäuse 1 eingedrungener Feuchtigkeit ist im Inneren des Gehäuses eine hygroskopische Substanz, wie beispielsweise Silika-Gel, angeordnet.

Bei den beiden in den Abbildungen Fig. 1 und 2 dargestellten Ausführungsformen eines Endoskops ist die hygroskopische Substanz in ein formbares Matrixmaterial eingebettet und als solchermaßen geformtes Bauteil in das Gehäuse 1 des Endoskops integriert. Das zum Beispiel durch Spritzgießen formbare, mit der hygroskopischen Substanz versetzte Matrixmaterial ist bei der ersten Ausführungsform gemäß Fig. 1 als O-Ring 8 ausgebildet, der in einer umlaufenden Ringnut 9 festlegbar ist.

Gemäß der in Fig. 2 dargestellten zweiten Ausführungsform ist das formbare Matrixmaterial als in das Gehäuse 1 eingesetzte zylindrische Hülse 10 ausgebildet.

Durch das Einbetten der hygroskopischen Substanz in das formbare Matrixmaterial ist es möglich, das hygroskopische Material an beliebigen Stellen und in unterschiedlichsten Geometrien im Gehäuse 1 anzuordnen, ohne hierfür besondere Montageelemente verwenden zu müssen. Das abriebfest und ortsfest einfach zu plazierende, die hygroskopische Substanz beinhaltende Matrixmaterial ist darüber hinaus schnell und einfach auszuwechseln.

Bei den dargestellten Ausführungsbeispielen ist es lediglich notwendig, die Okularmuschel 6 vom Handhabungsteil 4 des Gehäuses 1 abzuschrauben, um den O-Ring 8 oder die Hülse 10 zu entfernen und durch ein neues, trockens Bauteil zu ersetzen. Durch die Verwendung einer die Feuchtigkeitsbeladung durch eine Farbänderung anzeigenden hygroskopischen Substanz kann die Bedienbarkeit noch weiter vereinfacht werden, da der Beladungszustand der hygroskopischen Substanz mit Feuchtigkeit beispielsweise durch ein nicht dargestelltes spezielles Sichtfenster oder aber das Okulardeckglas 7 von außen bewertet werden kann.

Bei einem praktischen Versuch zur Herstellung eines O-Rings 8 gemäß Fig. 1 wurden als Material für den Matrixgrundwerkstoff 2K-additionsvernetzendes Silikon und als hygroskopische Substanz Silika-Gel (blau) verwandt.

### Bezugszeichenliste

- 1: Gehäuse
- 2: optisches System
- 3: Schaft
- 4: Handhabungsteil
- 5: Okulareinheit
- 6: Okularmuschel
- 7: Okulardeckglas
- 8: O-Ring
- 9: Ringnut
- 10: Hülse

## Patentansprüche

1. Optisches Instrument, insbesondere endoskopisches Instrument, mit einem Gehäuse (1), in dem mindestens ein optisches System (2) sowie eine hygroskopische Substanz angeordnet sind, wobei am Gehäuse (1) eine Okularmuschel (6) lösbar festgelegt ist,
**dadurch gekennzeichnet,**
**daß** die hygroskopische Substanz in ein formbares Matrixmaterial eingebettet ist, und daß das mit der hygroskopischen Substanz versetzte Matrixmaterial auswechselbar in die Okularmuschel (6) einsetzbar ist.

2. Optisches Instrument nach Anspruch 1, **dadurch gekennzeichnet, daß** das mit der hygroskopischen Substanz versetzte Matrixmaterial als in die Okularmuschel (6) einsetzbarer O-Ring (8) ausgebildet ist.

3. Optisches Instrument nach Anspruch 1, **dadurch gekennzeichnet, daß** das mit der hygroskopischen Substanz versetzte Matrixmaterial als in die Okularmuschel (6) einsetzbare zylindrische Hülse (10) ausgebildet ist.

4. Optisches Instrument nach mindestens einem der Ansprüche 1 bis 3, **dadurch gekennzeichnet, daß** das formbare Matrixmaterial im ausgehärteten Zustand elastisch und feuchtigkeitsdurchlässig ist.

5. Optisches Instrument nach mindestens einem der Ansprüche 1 bis 4, **dadurch gekennzeichnet, daß** das formbare Matrixmaterial ein Elastomer auf Silikon- und/oder Polyurethanbasis ist.

6. Optisches Instrument nach mindestens einem der Ansprüche 1 bis 5, **dadurch gekennzeichnet, daß** das mit der hygroskopischen Substanz versetzte Matrixmaterial durch Spritzgießen herstellbar ist.

7. Optisches Instrument nach mindestens einem der Ansprüche 1 bis 6, **dadurch gekennzeichnet, daß** die Feuchtigkeitsbeladung der hygroskopischen Substanz optisch anzeigbar ist.

8. Optisches Instrument nach Anspruch 7, **dadurch gekennzeichnet, daß** die hygroskopische Substanz die Feuchtigkeitsbeladung durch eine Farbänderung anzeigt.

9. Optisches Instrument nach mindestens einem der Ansprüche 1 bis 8, **dadurch gekennzeichnet, daß** die hygroskopische Substanz ein Silika-Gel oder eine poröse Keramik ist.

10. Optisches Instrument nach mindestens einem der Ansprüche 1 bis 8, **dadurch gekennzeichnet, daß** die hygroskopische Substanz aus einer Mischung verschiedener hygroskopischer Substanzen besteht.

## Claims

1. Optical instrument, in particular endoscopic instrument, having a housing (1) in which at least one optical system (2) and one hygroscopic substance are arranged, an eyepiece cup (6) being releasably attached to the housing (1),
**characterized in that** the hygroscopic substance is embedded in a mouldable matrix material and **in that** the matrix material to which the hygroscopic substance was added can be inserted into the eyepiece cup (6) such that it can be exchanged.

2. Optical instrument according to Claim 1, **characterized in that** the matrix material to which the hygroscopic substance was added is designed as an O-ring (8) which can be inserted into the eyepiece cup (6).

3. Optical instrument according to Claim 1, **characterized in that** the matrix material to which the hygroscopic substance was added is designed as a cylindrical sleeve (10) which can be inserted into the eyepiece cup (6).

4. Optical instrument according to at least one of Claims 1 to 3, **characterized in that** the mouldable matrix material is elastic and permeable to moisture in the cured state.

5. Optical instrument according to at least one of Claims 1 to 4, **characterized in that** the mouldable matrix material is a silicone - and/or polyurethane-based elastomer.

6. Optical instrument according to at least one of Claims 1 to 5, **characterized in that** the matrix material tc which the hygroscopic substance was added can be produced by injection moulding.

7. Optical instrument according to at least one of Claims 1 to 6, **characterized in that** the moisture loading of the hygroscopic substance can be displayed visually.

8. Optical instrument according to Claim 7, **characterized in that** the hygroscopic substance displays the moisture loading by means of a change in colour.

9. Optical instrument according to at least one of Claims 1 to 8, **characterized in that** the hygroscopic substance is a silica gel or a porous ceramic.

10. optical instrument according to at least one of Claims 1 to 8, **characterized in that** the hygroscopic substance comprises a mixture of different hygroscopic substances.

## Revendications

1. Instrument optique, en particulier instrument endoscopique, comportant un boîtier (1), dans lequel sont disposés au moins un système optique (2), ainsi qu'une substance hygroscopique, une bonnette d'oculaire (6) étant fixée de manière amovible sur le boîtier (1),
**caractérisé en ce que** la substance hygroscopique est enrobée dans un matériau de matrice formable et **en ce que** le matériau de matrice mélangé à la substance hygroscopique peut être mis en place de manière amovible dans la bonnette d'oculaire (6).

2. Instrument optique selon la revendication 1, **caractérisé en ce que** le matériau de matrice mélangé à la substance hygroscopique est réalisé sous la forme d'un joint torique (8), apte à être inséré dans la bonnette d'oculaire (6).

3. Instrument optique selon la revendication 1, **caractérisé en ce que** le matériau de matrice mélangé à la substance hygroscopique est réalisé sous la forme d'une douille (10) cylindrique, apte à être insérée dans la bonnette d'oculaire (6).

4. Instrument optique selon au moins l'une des revendications 1 à 3, **caractérisé en ce que** le matériau de matrice formable, à l'état durci, est élastique et perméable à l'humidité.

5. Instrument optique selon au moins l'une des revendications 1 à 4, **caractérisé en ce que** le matériau de matrice formable est un élastomère à base de silicone et/ou de polyuréthanne.

6. Instrument optique selon au moins l'une des revendications 1 à 5, **caractérisé en ce que** le matériau de matrice mélangé à la substance hygroscopique peut être réalisé par moulage par injection.

7. Instrument optique selon au moins l'une des revendications 1 à 6, **caractérisé en ce que** la charge d'humidité de la substance hygroscopique peut être constatée visuellement.

8. Instrument optique selon la revendication 7, **caractérisé en ce que** la substance hygroscopique indique la charge d'humidité par un changement de couleur.

9. Instrument optique selon au moins l'une des revendications 1 à 8, **caractérisé en ce que** la substance hygroscopique est un silicagel ou une céramique poreuse.

10. Instrument optique selon au moins l'une des revendications 1 à 8, **caractérisé en ce que** la substance hygroscopique est formée par un mélange de différentes substances hygroscopiques.
